# EUROPÄISCHE PATENTSCHRIFT

(11) **EP 4 280 983 B1**
(45) Veröffentlichungstag und Bekanntmachung des Hinweises auf die Patenterteilung: **04.03.2026**
(21) Anmeldenummer: 22700179.9
(22) Anmeldetag: 13.01.2022
(51) Int. Cl.: A61B 17/70, A61B 17/86, B22F 5/06, B22F 5/10, B24C 1/08, B24C 1/10, B33Y 10/00, B33Y 40/20, B33Y 80/00, C21D 9/00, C22F 1/18, C22C 1/04, C22C 14/00

(54) **SCHRAUBENELEMENT UND VERFAHREN ZUR ADDITIVEN HERSTELLUNG**
SCREW ELEMENT AND METHOD FOR ADDITIVE MANUFACTURE
ÉLÉMENT FILETÉ ET PROCÉDÉ DE FABRICATION ADDITIVE

(30) Priorität: 19.01.2021 DE 102021000210
(43) Veröffentlichungstag der Anmeldung: 29.11.2023
(73) Patentinhaber: Mimeo Medical GmbH, 70794 Filderstadt (DE)
(72) Erfinder: HEUER, Frank, 70794 Filderstadt (DE)
(74) Vertreter: Stolmár & Partner Patentanwälte PartG mbB
(86) Internationale Anmeldenummer: PCT/EP2022/050698
(87) Internationale Veröffentlichungsnummer: WO 2022/157074

(56) Entgegenhaltungen:
- EP-A1- 1 287 851
- EP-A1- 3 530 226
- CN-A- 110 882 414
- FR-A1- 3 052 659
- JP-A- H10 130 757
- US-A1- 2005 234 561
- US-A1- 2013 053 901
- US-A1- 2015 196 336
- US-A1- 2017 165 754
- US-A1- 2019 343 564

## Beschreibung

### Stand der Technik

Dem Stand der Technik sind verschiedene Osteosynthesevorrichtungen wie zum Beispiel Schraubenelemente zur Fixation von Knochen oder Knochenfragmenten bekannt. Hergestellt werden solche Knochenschrauben klassisch mit CNC-Fräs- und Drehmaschinen. Für die besonderen Knochengewinde-Geometrien und vor allem auch für die unterschiedlichen Durchmesser der Schraubenelemente sind spezielle Gewindeplatten bereitzustellen. Dies führt zu längeren Lieferzeiten und höheren Kosten. Der 3D-Druck bietet eine mögliche Alternative hierzu, denn es können sämtliche Geometrien ohne Sonderwerkzeuge, und damit auch ohne Wartezeit auf Sonderwerkzeuge, hergestellt werden. Eine deutlich größere Flexibilität in der Geometrie-Gestaltung ist ebenfalls möglich. Des Weiteren können weit mehr als einhundert Schraubenelemente gleichzeitig im 3D-Druck hergestellt werden, so dass sich generell ein erheblicher Kostenvorteil gegenüber den klassischen CNC-Herstellverfahren einstellt.

Ein wesentlicher Nachteil des 3D-Druck-Herstellverfahrens ist allerdings, dass die Oberflächen der gedruckten Teile eine natürliche Oberflächenrauigkeit aufweisen, die zwar optimal für das Anwachsen von Knochenzellen ist, aber dadurch auch zahlreiche Mikro-Kerben definiert werden. Diese Mikro-Kerben haben einen negativen Einfluss auf die Dauerfestigkeit der gedruckten Teile. Des Weiteren besitzen 3d-gedruckte Bauteile ein ausgeprägtes sprödes Materialverhalten, weshalb unterschiedliche Wärmebehandlungen vorgeschlagen werden, um diesem Materialverhalten entgegenzuwirken.

Aus dem Stand der Technik für Implantate ist zu entnehmen, dass im 3D-Druck-Verfahren Bandscheibenersatzimplantate (Cages) in Serie hergestellt werden, da hier die gedruckten Geometrien mechanisch so überdimensioniert sind, dass trotz der Mikro-Kerben die Mindestkriterien an die Dauerfestigkeit erreicht werden und von einer klinisch sicheren Situation für diese 3d-gedruckten-Cage-Implantate ausgegangen werden kann. Sollen allerdings Knochenschrauben im 3D-Druck hergestellt werden, sind deutlich gesteigerte Anforderungen an die Dauerfestigkeit zu stellen. Knochenschrauben sind einer andauernden und wiederkehrenden hohen Biegebelastung ausgesetzt. Deshalb müssen dafür geeignete Verfahrensschritte und diese in einer streng definierten Reihenfolge ausgeführt werden, damit einerseits das Material nicht spröde reagiert und sich die Mikro-Kerben entfernen lassen. Dem Stand der Technik fehlen jegliche Angaben zur Herstellung von 3d-gedruckten Knochenschrauben, insbesondere zur generellen Herstellung, der Nachbearbeitung und vor allem der Reihenfolgen von Nachbearbeitungsschritten damit sich eine klinisch sichere Dauerfestigkeit erreichen lässt.

Die US 2019/0343564 A1 offenbart eine Knochenschraube mit einem Schaft mit einer Wand, wobei die Wand einen kleinen Durchmesser aufweist, und mindestens einem Gewinde mit einer äußeren Gewindeform, wobei die Gewindeform eine Vielzahl von Gewinden umfasst, von denen mindestens eines der Gewinde einen ersten Abschnitt aufweist, der einen Kamm der Gewindeform hat, und einen zweiten Abschnitt, der von dem ersten Abschnitt eines der Gewinde zur Wurzel eines benachbarten Gewindes erstreckt, wobei der erste Abschnitt relativ zum zweiten Abschnitt eine feste Konfiguration aufweist Abschnitt, wobei sich der zweite Abschnitt kontinuierlich von einer Wurzel des einen der Fäden bis zur Wurzel des benachbarten eines der Gewinde erstreckt. Die Wand des Gewindes kann eine Lattice-Struktur aufweisen.

Die FR 3 052 659 A1 offenbart ein Verfahren zur Herstellung von mindestens einem Implantatabutment einer Zahnprothese, wobei ein Implantatabutment einen Einsatz in einem unteren Teil aufweist, der eine Verbindung hat, die so konfiguriert ist, dass sie das Abutment Pfeiler mit einem Implantat verbindet. Das Verfahren umfasst ferner einen Körper im oberen Teil, der so konfiguriert ist, dass er eine Krone trägt, wobei das Verfahren weiter einen Schritt zur Herstellung eines Implantatabutmentrohlings aufweist, einen Schritt zur spezifischen Herstellung des Körpers des Implantatabutments, einen Schritt zur Herstellung des Körpers durch Herstellung auf einer Platte für die additive Fertigung, beginnend mit dem ersten Teil des Schafts, beginnend mit einem Scheitelpunkt des Körpers, und einen Schritt der Herstellung eines Einsatzrohlings in einer Verlängerung des Körpers, umfassend einen Unterschritt der Herstellung eines Verbindungsrohlings durch additive Fertigung auf der gleichen additiven Fertigungsplatte, und einen Schritt zur Herstellung der Anschlusstechnik, der mindestens einen Schritt des Funkenerodierens des Anschlussteilrohlings durch Senken von mindestens einer Elektrode auf den Steckverbinderrohling, wobei die mindestens eine Elektrode in den Steckverbinderrohling (33') eingeprägt wird, wobei eine Elektrode eine Vertiefung aufweist, die eine Form darstellt, die komplementär zur Anschlusstechnik des herzustellenden Einsatzes ist.

Die US 2013/0053901 A1 offenbart eine kanülierte Knochenankerbaugruppe umfassen einen Knochenanker, ein Aufnahmeelement zum Aufnehmen eines Wirbelsäulenfixierungselements, das mit dem Knochenanker gekoppelt 'wird, und einen Verschlussmechanismus zur Fixierung des Wirbelsäulenfixierungselements in Bezug auf das Aufnahmeelement. Der Knochenanker umfasst einen distalen Schaft mit einem ersten Gewindeabschnitt proximal zu einem proximalen Kopf, einen zweiten Gewindeabschnitt proximal und benachbart zu dem ersten Gewindeabschnitt, und einen dritten Gewindeabschnitt, der proximal dazu und benachbart zu dem zweiter Gewindeabschnitt liegt. Der erste Gewindeabschnitt hat einen konstanten Haupt- und Nebendurchmesser. Der zweite Gewindeabschnitt hat eine sich verjüngende Haupt- und Nebendurchmesser. Der dritte Gewindeabschnitt mit einem sich verjüngenden Hauptdurchmesser und einem konstanten Nebendurchmesser.

Die US 2015/0196336 A1 offenbart ein orthopädisches Befestigungselement mit einem Kopf und einem Schaft. Der Schaft hat einen vorderen Endabschnitt, der an eine distale Spitze angrenzt, und einen hinteren Endabschnitt, der an einen Kopf angrenzt. Zwischen dem vorderen und dem hinteren Endabschnitt befindet sich ein Zwischenabschnitt. Am Zwischenabschnitt vergrößert sich der Durchmesser des Schaftes. Der vordere Endabschnitt hat ein erstes Gewinde, das eine oder mehrere selbstschneidende Schneidnuten aufweist, die sich neben der distalen Spitze und durch eine Vielzahl der ersten Gewinde erstrecken. Der Zwischenabschnitt hat ein zweites Gewinde, das sich vom vorderen Endabschnitt in Richtung des Kopfes erstreckt. Die zweiten Gewindegänge des Zwischenabschnitts haben einen größeren Außendurchmesser als die ersten Gewindegänge im vorderen Endabschnitt. Der Zwischenabschnitt hat eine oder mehrere Schneidnuten, die jeweils an einem vorderen Übergang am vorderen Endabschnitt und an einem hinteren Übergang verlaufen.

Die 2005/0234561 A1 offenbart eine verschleißfeste orthopädische Vorrichtung aus einer Titanlegierung. Außerdem wird ein Verfahren zur Herstellung einer verschleißfesten orthopädischen Vorrichtung aus Titanlegierung durch tiefe Diffusion von Sauerstoff in die Vorrichtung aus Titanlegierung bereitgestellt. Ein Verfahren zum Härten einer Vorrichtung aus Titan durch tiefes Eindiffundieren von Sauerstoff in die Vorrichtung aus Titanlegierung wird bereitgestellt.

### Darstellung der Erfindung

Die Aufgabe wird anhand des hier vorgestellten erfindungsgemäßen Schraubenelements und dessen Herstellverfahren gemaß den Ansprüchen 1 und 9 gelöst.

Für die Herstellung soll ein additives Herstellverfahren zum Einsatz kommen. Additive Fertigungen von metallischen Legierungen, oder auch 3D-Druck genannt, greifen auf das Laser- oder Elektronenstrahl-Schmelzverfahren zurück. Als Material eignen sich alle metallischen Legierungen, die als orthopädischer Implantatwerkstoff bekannt und akzeptiert sind. Dazu gehören beispielsweise Titan-, Cobalt-Chrom und Edelstahllegierungen. Vorzugsweise soll die Titan-Legierung Ti6Al4V zum Einsatz kommen. Für den 3D-Druck werden Rohmaterial-Partikel oder -Pulver mit einer definierten Korngröße verwendet. Mit der Korngröße lassen sich die Bauteilgenauigkeit und der Schichtabstand bei der Herstellung vorgeben.

Hersteller von 3D-Druck-Maschinen schlagen für das beste Ergebnis bzgl. der Oberflächengüte und Genauigkeit eine schräge Platzierung im 3D-Druck-Bauraum vor. Eine schräge Platzierung im Bauraum würde verlangen, dass das erfindungsgemäße Schraubenelement (1) schräg im 3D-Drucker aufgebaut werden müsste und eine Vielzahl von seitlichen Ausleger-Elementen oder Stützelementen vorgesehen sein müssten, damit ein optimales Druckergebnis erzielt werden kann. Dies erzeugt zwar das qualitativ beste Ergebnis, aber es ist kosten-ineffizient. Einerseits passen deutlich weniger Schraubenelemente in den druckbaren Bauraum und anderseits können seitlich befestigte Ausleger-Elemente nur manuell, d.h. händisch und damit in einer Serienherstellung mit großer Stückzahl nur kosten-ineffizient entfernt werden. Damit steht dem besten Druckergebnis eine entsprechende Kosten-Ineffizienz in der Serien-Herstellung entgegen.

Zur effektiven Nutzung des Bauraums und damit gleichzeitig eine Maximierung der Anzahl herstellbaren Schraubenelemente (1) erreicht wird, ist es von Vorteil, wenn die Schraubenelemente (1) stehend hergestellt werden könnten. Das bringt allerdings Einschränkungen bzgl. der herstellbaren Merkmale und druckbaren Genauigkeiten mit sich. Damit Schraubenelemente (1) dennoch stehend und kosteneffizient gedruckt werden können, müssen verschiedene geometrische Merkmale eingehalten und vorgesehen werden.

Für das erfindungsgemäße Schraubenelement (1), sind raumzuweisende Koordinatenreferenzen definiert, wie zum Beispiel die proximale Richtung (101), die distale Richtung (102), die sich entlang einer Zentralachse (103) ausdehnen. Von der Zentralachse (103) nach außen abgehend definiert sich die radiale Ausbreitung (104) (Fig. 1 und Fig. 2). Das erfindungsgemäße Schraubenelement (1) ist für die Fixation von Knochenkomponenten und Knochenfragmenten geeignet und besteht aus einem Schaft (11) mit einem Knochengewinde (12) und eine sich am Schaft (11) entlang erstreckende longitudinale Zentralachse (103) und das Schraubenelement (1) zusätzlich einen Kopf (10), einen Halsbereich (20) aufweist und eine Werkzeugansatzstelle (90), die im Kopf (10) bereitgestellt wird (Fig. 4).

In der bevorzugten Ausführungsform sind am proximalen Ende des Kopfes (10, 109) mindestens drei Ausleger-Elemente (13) befestigt und diese Ausleger-Elemente (13) ragen in proximaler Richtung (101) über das proximale Ende des Kopfes (10, 109) hinaus. Dabei sind sie hauptsächlich entlang der longitudinalen Zentralachse (103) angeordnet. Die Ausleger-Elemente (13) sind vorzugsweise einstückig mit dem proximalen Ende des Kopfes (10, 109) verbunden. Die Ausleger-Elemente (13) können dabei eine beliebige Form oder Geometrie besitzen. Beispielsweise können sie aus Platten-, Draht-, Balken- oder Säulen-Elemente bestehen oder einer Kombination aus diesen zusammengefügt sein. Dabei können die Ausleger-Elemente mindestens teilweise eine zusätzliche radiale Beabstandung zum Schraubenelement herstellen. Die Ausleger-Elemente können mit einanderweitere Verbindungen aufweisen, um die Ausleger-Elemente als Verbund zu gestalten. Des Weiteren ist auch eine ringartige oder polygonartige Anordnung von ein oder mehr Formationen denkbar. Die Aufgabe der Ausleger-Elemente ist es das Schraubenelement (1) während dem 3D-Druck-Vorgang von einer Grundplatte des 3D-Druckers her zu beabstanden und gleichzeitig zu halten. Die freien Enden (17) der Ausleger-Elemente (13) beschreiben eine Ebene (108) , so dass die Schraubenelemente (1) mit Hilfe dieser Ebene (108) während der additiven Herstellung eine Stabilität gegenüber einem Verkippen erzielt wird. Für das erfindungsgemäße Schraubenelement (1) wird eine Baurichtung (105) für den 3D-Druck definiert, die in etwa der Richtung der Zentralachse (103) entspricht, und von proximal (101) nach distal (102) verläuft und die Baurichtung (105) der Flächennormalen der Ebene (108) entspricht. Erst in dieser Kombination dieser Merkmale ist eine stehende Fertigung überhaupt erst möglich.

Damit nicht die vollständige Außenfläche des Kopfbereichs (10) mit Ausleger-Elemente (13) versehen sein muss, ist es vorteilhaft, wenn die Werkzeugansatzstelle (90) in proximaler Richtung (101) offen ist und in einer konzentrischen kegelartigen Ausnehmung (94) mündet und einen in etwa rechtwinkligen Konuswinkel besitz. Somit ist es ausreichend, wenn die Ausleger-Elemente (13) auf einer proximalen Ringstruktur (95, 109) entlang des Durchmessers der Werkzeugansatzstelle vorgesehen sind.

Aus dem gleichen Grund ist es auch wichtig, dass die Werkzeugansatzstelle (90) nach distaler Richtung (102) von einer Wandung (93) begrenzt ist und diese Wandung (93) als Konus in zunehmender distaler Richtung (102) nach radial innen verläuft und der durch die Wandung gebildete Konuswinkel kleiner als 120° ist. Vorzugsweise besitzt diese Wandung (93) einen in etwa rechtwinkligen Konuswinkel. Damit ist diese Fläche (93) unter Berücksichtigung der definierten Baurichtung (105) im 3D-Druck herstellbar. Somit können auch hier Ausleger-Elemente für den Boden der Werkzeugansatzstelle eingespart werden. Das Entfernen etwaiger Ausleger-Elemente von dieser Bodenfläche (93) wäre eine große Herausforderung, da diese Bodenfläche (93) sehr schlecht für die Nachbearbeitung erreichbar ist. Vorteilhaft ist es auch wenn die Zahnprofile (91) ebenfalls in dieser Konus-Fläche (93) münden, damit diese Mündungsstellen im 3D-Druck-Verfahren herstellbar sind. Eine solche konus-artige Wandung (93) am Grund der Werkzeugansatzstelle (90) würde sich mit CNC-Herstellverfahren nicht oder nur unter höchsten technischen Aufwand erzeugen lassen.

In einer bevorzugten Ausführungsform wird ein Schraubenelement (1) für die Fixation von Knochenkomponenten und Knochenfragmenten beschrieben, welcher aus einem Schaft (11), einem Halsbereich (20) und einen in proximaler Richtung (101) befindlichen Kopf (10), sowie in distaler Richtung (102) befindlicher Spitze (60) besteht. Der Kopf (10) ist vorzugsweise als Linse, Schrägkopf oder als Kugelkopf ausgebildet. Es sind aber auch eine Zusammensetzung aus unterschiedlichen Rundungen und Flächen denkbar. Hauptmerkmal des Kopfes ist es, dass der Kopf (10) einen größeren Außendurchmesser als der Halsbereich (20) aufweist. Vorzugsweise besitzt der Knochenanker eine Werkzeugansatzstelle (90), die zur Einleitung eines Drehmoments geeignet ist. Zur minimal-invasiven Versorgung ist es vorteilhaft, wenn der Knochenanker eine vollständig durchquerende Kanülierungsöffnung (80) besitzt, durch die ein chirurgischer Führungsdraht geführt werden kann.

Als Schraubenelement (1) kommen vorzugsweise Knochenschrauben zum Einsatz, die mit einem Knochen verschraubbar sind. Es sind aber auch Haken, Klemmen, Nägel und andersartig gestaltete Knochenanker anwendbar. In dem hier aufgeführten Beispiel eines Schraubenelements (1), wird eine Knochenschraube mit einem Schaft (11) und einen am Schaft befindlichen Knochengewinde (12) vorgestellt. Das Gewindegewinde (12) kann proximal zumindest abschnittsweise eine feinere Verzahnung (30) aufweisen, welches für einen härteren Kortikal-Knochen besser geeignet ist. Vorteilhaft ist ein nach distal spitz zulaufendes Gewinde (60) mit einer Schnittkante (61) an der Knochenankerspitze (60), damit sich der Schraubenelement (1) beim Einschrauben selbstschneidend in den Knochen ziehen kann.

Dabei ist es vorteilhaft wenn das Schraubenelement (1) dadurch charakterisiert ist, dass das Außengewinde (12) in einen an den Halsbereich (20) angrenzenden und in distaler Richtung (102) erstreckenden proximalen Gewindebereich (30), und ein sich daran angrenzender distaler Gewindebereich (50), und wiederrum daran angrenzender distaler Spitzenbereich (60) einteilen lässt, und der distale Gewindebereich (50) in den proximalen Gewindebereich (30) in einer Übergangszone (40) ineinander übergehen, und der proximale Gewindebereich (30) mindestens einen zusätzlichen Gewindegang (31, 32) ausbildet, welcher innerhalb der Übergangszone (40) mindestens eine Schneidkante (41) ausbildet.

Durch diese Schneidkante (41) wird eine vorschneidende Wirkung erzielt und keine Raumverdrängung im Knochen verursacht. Dies ist insbesondere bei schwächerem Knochen von klinischem Vorteil, denn es kann das Risiko einer Frakturen während der Implantation reduzieren.

Weiterhin vorteilhaft ist, wenn wenigstens eine der Schneidkanten (41, 61) planar ist und hauptsächlich in radialer Richtung (104) ausgerichtet ist. Alternativ ist auch eine konkave oder konvexe Fläche zur Erzeugung der jeweiligen Schneidkante denkbar.

Als Knochengewinde sind unterschiedliche Gewindezahnverläufe und Anordnungen denkbar. Beispielsweise kann ein Gewinde mit einem Zahn am distalen Bereich auf ein Doppel- oder Dreifach-Gewinde in den proximalen Bereich übergehen. Es ist auch ein im distalen Bereich Doppel-Gewinde vorsehbar, welches in promxialer Richtung (101) in ein Vierfach- oder Sechsfach-Gewinde übergeht. Zur Vereinfachung sämtlicher Darstellungen wurde die bevorzugte Ausführungsform mit einem Doppelgewinde im distalen Bereich (50) und einem Vierfachgewinde am proximalen Bereich (30) abgebildet.

Bei schwachem Knochen, wie beispielsweise bei einer Osteopenie oder Osteoporose, kann es notwendig sein, den Knochenanker zusätzlich zu augmentieren. Dies kann mit Knochenzement erfolgen. Knochenzement ist vorzugsweise ein Polymer, welches aus mindestens zwei Komponenten vermischt und im flüssigen bzw. pasten-ähnlichem Zustand injiziert wird. der Knochenzement härtet nach wenigen Minuten im Knochen zu einem Kunststoff aus und verbindet sich mit der schwamm-artigen Knochenstruktur. Angewandt wird meist ein Polymethylmethacrylat-Zement. Alternativ sind auch andere Medien für die Abgabe durch den Knochenanker denkbar. Es ist denkbar, dass alternative Medien, wie beispielsweise pharmazeutisch wirkende Medien, oder Medien mit Zellen, Nährstoffe, oder Medien die als Erbinformationsträger dienen, oder Impfstoffe durch den Knochenanker verabreicht werden.

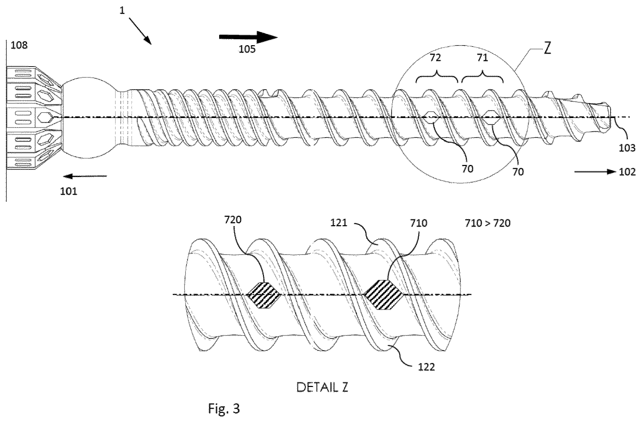

Optional besitzt die Kanülierung (80) mindestens eine oder mehr seitlich abgehende und mit der Kanülierung kommunizierende Öffnungen (70) (Fig. 3). Vorzugsweise sind die Öffnungen in Umfangsrichtung in ring-artiger Formation (71 oder 72) angeordnet. Bei mehr als einer in Umfangsrichtung ring-artiger Öffnungs-Formation (71 und 72), haben die Öffnungen je Formation unterschiedliche Öffnungsquerschnittsflächen (710, 720). Bei in einen Knochen eingeschraubten Knochenanker (1), kommunizieren die seitlichen Öffnungen von der Hohlkammer (80) aus mit dem umgebenden Knochengewebe. Sie sind dazu geeignet, dass die Flüssigkeit, die in den Knochenanker (1) injiziert wird, durch die seitlichen Öffnungen in das umliegende Gewebe abgegeben wird. Eine unterschiedliche Querschnittsfläche der Öffnungs-Formationen (710, 720) hat den Vorteil, dass aufgrund des lokalen Druckunterschieds innerhalb der Flüssigkeit, durch alle Öffnungen (71, 72) ein ähnlicher Volumenstrom generiert wird. Dies wird dadurch erreicht, dass die Öffnungen (72), die näher zur proximalen Richtung (101) liegen, eine kleinere Querschnittsfläche (720) besitzen als die Öffnungen (710) der Formation (71), die weiter distal liegen.

Für den 3D-Druck besonders vorteilhaft ist, wenn diese seitlichen Öffnungen (70, 71, 72) als Polygon (700) vorgesehen sind. Herkömmlich werden solche seitlichen Öffnungen (70) konzentrisch ausgebohrt. Beim 3D-Druck würden konzentrische Öffnungen kleine Überhänge entstehen und daraus resultieren sogenannte Dross-Formationen (d.h. Miniatur-Tropfstein-ähnliche Formationen). Dies würde eine in der Serienfertigung kostspielige manuelle Nachbearbeitung erfordern. Eine Alternative, und damit bevorzugte Ausführungsform, bieten Polygone. Die schräg zulaufen Flächenelemente eines Polygons bilden einen Dachähnlichen Aufbau. Schrägen mit einem Konus-Winkel zueinander von etwa 90° sind problemlos im Druckverfahren ohne Ausleger-Elemente oder Dross-Formationen herzustellen.

Mit dem 3D-Druck ist es auch möglich unterschiedlichen Rauigkeiten auf der Oberfläche des Schraubenelements (1) vorzusehen. So wird erfindungsgemäß auf den Gewinde-Flächen (121) der Gewindeflanken (12), die nach proximaler Richtung gerichtet sind, eine größere Oberflächenrauigkeit vorzusehen, als auf den Gewinde-Flächen (122) derselben Gewindeflanken, die nach distal gerichtet sind (Fig. 3). Eine höhere Rauigkeit auf den Gewinde-Flächen (121) in proximaler Projektionsrichtung hat den Vorteil, dass eine höhere Reibung zwischen Knochen und Schraubenelement in Auszugsrichtung erzeugt wird und das Schraubenelement eine signifikant höhere Auszugsfestigkeit erhält. Glattere Gewinde-Flächen (122) in distaler Projektionsrichtung haben den Vorteil, dass sich das Schraubenelement (1) dann trotzdem leicht in den Knochen einschrauben lässt.

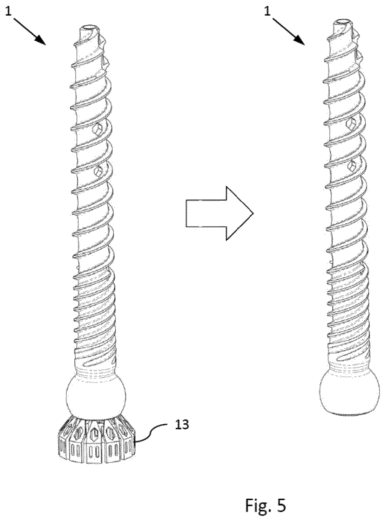

Verfahren zum Herstellen eines Schraubenelements (1) nach einem der vorhergehenden Ansprüche, umfassend folgende Schritte:
a. Bereitstellen von Rohmaterial-Partikel einer Ti6A14V-Legierung.
b. Additives Aufbauen des erfindungsgemäßen Schraubenelements (1) mit Hilfe eines 3D-Druck-Verfahrens, welches die Rohmaterial-Partikel drei-dimensional miteinander zusammenfügt, so dass am proximalen Ende des Kopfes mindestens drei Ausleger-Elemente befestigt sind, deren freie Enden eine Ebene beschreiben, und so dass das Außengewinde des Schraubenelements mindestens eine Gewinde-Fläche besitzt, die von der Ebene weggerichtet ist, und mindestens eine Gewinde-Fläche besitzt, die dieser Ebene zugerichtet ist, und die der Ebene zugerichtete Gewinde-Fläche eine größere Rauigkeit aufweist als die der Ebene weggerichtete Gewinde- Fläche.
c. Spannungsarmglühen des Schraubenelements (1) bei einer Temperatur von mindestens 500°C, jedoch nicht höher als 840°C, mit einer Haltezeit von wenigstens einer Stunde, aber kürzer als 6 Stunden.
d. Entfernen der Ausleger-Elemente (13) vom proximalen Kopfbereich (109) (Fig. 5).
e. Zweite Wärmebehandlung des Schraubenelements (1).
f. Entfernen unvollständig gefügter Rohmaterial-Partikel.

Für das Spannungsarmglühen ist es von Vorteil, wenn beim Spannungsarmglühen des Schraubenelements (1) eine Temperatur von vorzugsweise zwischen 550°C und 800°C, vorzugsweise zwischen 550°C und 750°C, vorzugsweise zwischen 600°C und 720°C, für die Dauer der Haltezeit eingestellt ist.

Das Entfernen der Ausleger-Elemente (13) kann manuell oder auch maschinell durchgeführt werden. Hierfür eignen sich beliebige zerspanende oder trennende Verfahren, wie beispielsweise Schleifen, Fräsen, Drehen oder auch Senk- und Drahterodieren.

Für die zweite Wärmebehandlung des Schraubenelements (1) sind drei unterschiedliche Verfahren zielführend. Mit der zweiten Wärmebehandlung wird das Materialgefüge bzgl. des ursprünglich sprödem Verhaltens so optimiert, dass sich eine gewissen Materialzähigkeit einstellen lässt. Hierfür gibt es unterschiedliche Temperaturbereiche und Umgebungsbedingungen, bei denen sich die besten Materialeigenschaften einstellen lassen. Eine Option der zweiten Wärmebehandlung des Schraubenelements (1) ist die Erzeugung einer Argon-Atmosphäre, bei einer Temperatur zwischen 900°C und 940°C, vorzugsweise zwischen 910°C und 930°C, und das Schraubenelement (1) einem Druck von mindestens 900bar, vorzugsweise von mindestens 950bar ausgesetzt ist, und für die Temperatur und Druck eine Haltezeit von mindestens einer Stunde aber weniger als 4 Stunden eingehalten wird. Eine weitere Option der zweiten Wärmebehandlung des Schraubenelements (1) ist die Erzeugung einer Argon-Atmosphäre bei einer Temperatur von wenigstens 920°C aber weniger als 1050°C, bei einer Haltezeit von mindestens einer Stunde aber weniger als 4 Stunden. Eine weitere Option der zweiten Wärmebehandlung des Schraubenelements (1) ist die Erzeugung einer Temperatur von wenigstens 820°C aber weniger als 920°C, und für die Dauer einer Haltezeit von mindestens einer Stunde aber weniger als 4 Stunden aufrechtgehalten wird.

Die Wärmebehandlungen (Spannungsarmglühen und die zweite Wärmebehandlung zur Optimierung des Materialgefüges) allein ist nicht ausreichend für die Erhöhung einer Dauerfestigkeit. Eine entsprechende Dauerfestigkeit stellt sich nur ein, wenn das Material die richtigen Eigenschaften ausweist und die Oberflächen frei von Mikro-Kerben sind. Deshalb beinhaltet das Verfahren zur Herstellung des Schraubenelements den Verfahrensschritt "Entfernen unvollständig gefügter Rohmaterial-Partikel". Dabei es ist vorteilhaft, wenn zwei unterschiedliche abtragende Verfahren zum Einsatz kommen, die sich gegenseitig ergänzen. Das Entfernen unvollständig gefügter Rohmaterial-Partikel soll deshalb Folgendes umfassen:
g. Entfernen unvollständig gefügter Rohmaterial-Partikel mit Hilfe eines mechanisch-abtragenden Verfahrens, und
**h.** Entfernen unvollständig gefügter Rohmaterial-Partikel mit Hilfe eines chemisch-abtragenden Verfahrens.

Für die Automatisierung der Serienherstellung kann das mechanisch-abtragende Verfahren mit Hilfe eines Strahlmittels, bei einem Mindestapplikationsdruck von 2bar, und einer Mindestapplikationsdauer von 5min durchgeführt wird, wobei scharfkantiges Strahlmittel, wie beispielsweise Korund, zum Einsatz kommt, und das Strahlmittel eine Korngröße zwischen 0,05mm und 0,25mm, vorzugsweise jedoch eine Korngröße zwischen 0,07mm und 0,15mm aufweist. Alternativ kann das mechanisch-abtragende Verfahren auch mit Hilfe eines Grobgleitschleifen- oder Vibrationsschleifverfahren durchgeführt werden.

Das chemisch-abtragende Verfahren wird mit Hilfe eines chemischen Beiz- oder Ätzvorgangs durchgeführt. Ein chemisches Verfahren hat den Vorteil, dass auch Konturen und geometrische Merkmale, die nicht von außen erreichbar oder zugängig sind, trotzdem nachbearbeitet bzw. abgetragen werden können. Somit kann beispielsweise auch die Innenkontur der Kanülierung (80) von unvollständig gefügten Partikeln befreit werden. Das chemisch-abtragende Verfahren kann elektro-galvanisch und/oder durch mechanisch induzierte Schwingungen unterstützt werden.

Die Dauerfestigkeit bei biege-belasteten Bauteilen kann zusätzlich gesteigert werden, indem Druckeigenspannungen an den Oberflächen erzeugt werden. Dies kann durch geeignetes Strahlmittel erzielt werden. Deshalb ist es vorteilhaft, wenn zum Abschluss des Verfahrens die Oberflächen des Schraubenelements (1) mit Hilfe eines Strahlmittels verfestigt werden, wobei ein Mindestapplikationsdruck von 2bar, und einer Mindestapplikationsdauer von 5min eingehalten werden, wobei kugelartiges Strahlmittel zum Einsatz kommt, wie beispielsweise Keramikkugeln, und das Strahlmittel eine Korngröße zwischen 0,05mm und 0,25mm, vorzugsweise jedoch eine Korngröße zwischen 0,07mm und 0,15mm aufweist.

### Kurze Beschreibung der Zeichnungen zeigen

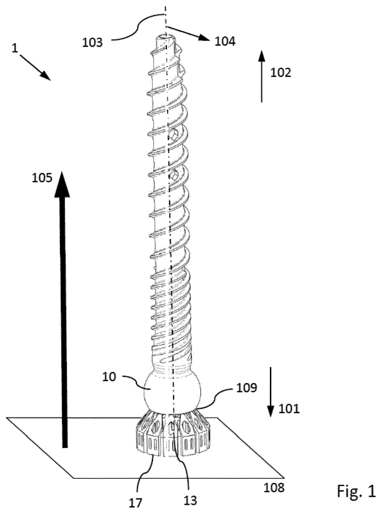

Fig. 1 eine Schrägansicht des erfindungsgemäßen Schraubenelements in stehender Orientierung,

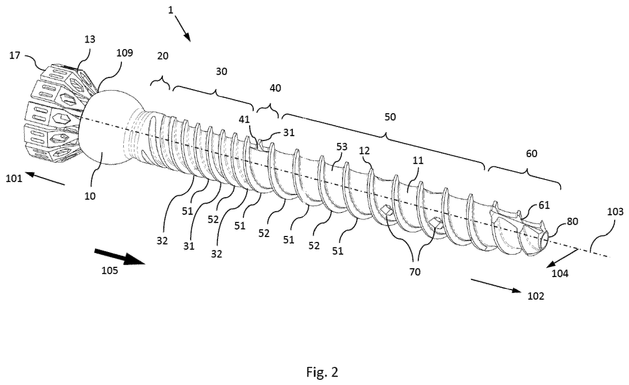

Fig. 2 eine Schrägansicht von distal.
Fig. 3 eine Seitensicht des erfindungsgemäßen Schraubenelements, sowie eine Detaildarstellung der seitlichen Öffnungen und Gewindeflanken.

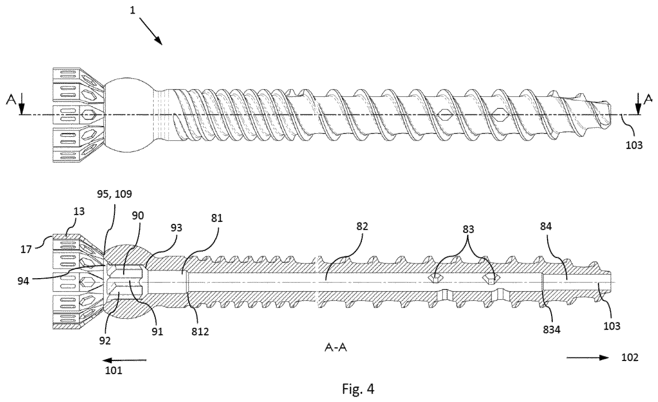

Fig. 4 Seitenansicht und dazu gehöriger Schnittansicht durch den erfindungsgemäßen Knochenanker,
Fig. 5 ein Arbeitsschritt während des Herstellverfahrens.

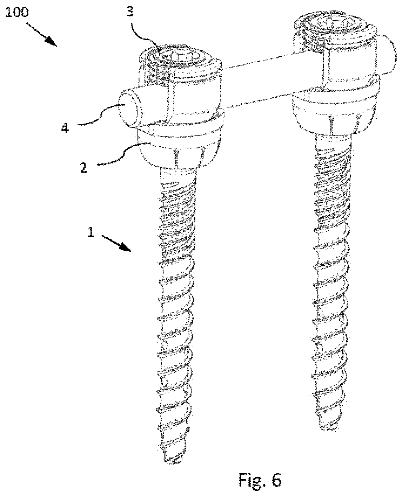

Fig. 6 zwei erfindungsgemäße Schraubenelemente im Verbund mit u-förmigen Gabelköpfen montiert mit einem Verbindungsstab.

### Beschreibung der bevorzugten Ausführungsformen

Der Fig. 1 ist zu entnehmen, dass die Baurichtung (105) des erfindungsgemäßen Schraubenelements (1) in etwa der Richtung der Zentralachse (103) entspricht, und von proximal (101) nach distal (102) verläuft. Des Weiteren entspricht die Baurichtung (105) einer Flächennormalen (108). Die Vorteile wurden bereits eingangs beschrieben.

Fig. 2 zeigt eine Ausführungsform eines Schraubenelements (1) bestehend aus einen Kopfbereich (10), einen Halsbereich (20) und einen Schaft-Bereich (11) mit Knochengewinde (12). Weiterhin ist er erkennbar, dass das Außengewinde (12) in einen an den Halsbereich (20) angrenzenden und in distaler Richtung (102) erstreckenden proximalen Gewindebereich (30), und ein sich daran angrenzender distaler Gewindebereich (50), und wiederrum daran angrenzender distaler Spitzenbereich (60) einteilen lässt, und der distale Gewindebereich (50) in den proximalen Gewindebereich (30) in einer Übergangszone (40) ineinander übergeht, und der proximale Gewindebereich (30) mindestens einen zusätzlichen Gewindegang (31, 32) ausbildet, welcher innerhalb der Übergangszone (40) mindestens eine Schneidkante (41) ausbildet.

Eine weitere Schneidkante (61) ist an dem distalen Spitzenbereich (60) ausbildet. Optimalerweise verläuft die jeweilige Schneidkante (41, 61) hauptsächlich in radialer Richtung planar. Andere Flächengeometrien mit konvexen oder konkaven Bereichen sind ebenfalls denkbar. Alternativ sind auch in Umfangsrichtung wiederkehrende Muster, die eine vorschneidene Wirkung haben, wie beispielsweise Riffelungen oder Zähne, denkbar.

Fig. 2 illustriert in einer Ausführungsform ein Schraubenelement (1) welches im distalen Bereich (50) zwei voneinander getrennte Gewindezähne (51 und 52) ausbildet. Dies ist ein sogenanntes Doppelgewinde, wobei im Vergleich zu einem Einfachgewinde bei gleicher Gewindezahn-Anzahl eine größere Steigung erzielt wird. Das reduziert die notwendige Anzahl von Umdrehungen, um ein solches Schraubenelement (1) zu implantieren. Zwischen den Gewindegängen befindet sich der Gewindekern oder die Gewinde-Täler (53). Im proximalen Bereich (30) wird zwischen den distalen Gewindegängen (51, 52) jeweils ein zusätzlicher Gewindezahn (31, 32) vorgesehen. Die proximalen Gewindegänge (31, 32) besitzen dabei dieselbe Steigung, wie die distalen Gewindegänge (51 und 52). In dem Übergangsbereich (40) werden zwei Schneidkanten (41, 42) ausgebildet, wobei die zweite Schneidkante (42) aufgrund der Ansicht nicht darstellbar ist. Sie ist der (hier nicht sichtbare) Start für den zweiten proximalen Gewindegang (32). Die Schneidkanten (41, 42) haben den großen klinischen Vorteil, dass mit solchen Schraubenelementen (1) zukünftig Frakturen während der Implantation verhindert werden können. Ist alternativ ein anderes Gewinde vorgesehen, erhöhen oder reduzieren sich die Anzahl der Schneidkanten dementsprechend.

In Fig. 3 ist eine bevorzugte Ausführungsform eines Schraubenelements (1) dargestellt, bei dem die seitlichen Fenestrationsöffnungen (70) als Polygon ausgestaltet sind. Außerdem darstellt ist, dass die seitlichen Öffnungen (70) in Umfangsrichtung in ring-artiger Formation (71 und/oder 72) angeordnet sind und bei mehr als einer in Umfangsrichtung ring-artiger Formation (71 und 72) die Öffnungen (70) je Formation unterschiedliche Öffnungsquerschnittsflächen (710, 720) aufweisen. Optimalerweise ist die Öffnungsquerschnittsfläche (710) der distalen Formation (71) größer als die Öffnungsquerschnittsfläche (720) der proximalen Formation (72).

Fig. 4 offenbart eine Schnittansicht des Schraubenelements (1). Zu erkennen ist, das Innere des Kopfbereichs (10) und die durchgängige Kanülierung (80). Hauptmerkmal des Kopfes ist es, dass der Kopf (10) einen größeren Außendurchmesser als der Halsbereich (20) aufweist. Vorzugsweise besitzt der Knochenanker eine Werkzeugansatzstelle (90), welche zur Einleitung eines Drehmoments geeignet ist. Das Drehmoment zum Einschrauben des Knochenankers kam damit direkt über die Werkzeugansatzstelle eingeleitet werden. Diese Werkzeugansatzstelle kann ein beliebiges Profil (91,92) besitzen, wie beispielsweise einen Vielzahnrund, Innensechskant, Kreuzschlitz, einen einfachen Schlitz oder eine andersartig gestaltete Verzahnung aufweist. In der bevorzugten Ausführungsform befindet sich die Werkzeugansatzstelle (90) am proximalen Ende (101) und ist nach distaler Richtung (102) von einer Wandung (93) begrenzt. Diese Wandung (93) ist dabei als Schräge bzw. Konus ausgeformt, die in zunehmender distaler Richtung (102) nach radial innen verläuft und der durch die Wandung gebildete Konuswinkel ist kleiner als 120°. Optimalerweise ist der Konuswinkel in etwa rechtwinklig. Des Weiteren ist erkennbar, dass die Werkzeugansatzstelle (90) in proximaler Richtung (101) offen ist und in einer konzentrischen kegelartigen Ausnehmung (94) mündet und einen in etwa rechtwinkligen Konuswinkel besitzt.

In Fig. 4 ebenfalls dargestellt, ist der Verlauf der Kanülierungsöffnung (80). Vorteilhaft ist es, wenn proximal ein Abschnitt (81) mit einem etwas größeren Durchmesser vorgesehen ist, in dem eine Applikationskanüle eingeführt werden kann. Daran angrenzend befindet sich der mittlere Teil der Kanülierung (82) mit einem Durchmesser. Die seitlichen Öffnungen (70) münden in die Kanülierung (82) durch entsprechende Mündungsstellen (83). Vorteilhaft ist es, wenn nach distal (102) der Kanülierungsdurchmesser in einem distalen Kanülierungsabschnitt (84) reduziert ist. Die Übergänge (812, 834) zwischen den unterschiedlichen Kanülierungsdurchmessern besitzen optimalerweise einen Konuswinkel von weniger als 120°, vorzugsweise sind sie in etwa rechtwinklig damit auch sie für den 3D-Druck optimal hergestellt werden können.

Fig. 5 stellt einen Arbeitsschritt des Herstellverfahrens dar, bei dem die Ausleger-Elemente (13) entfernt werden.

Fig. 6 illustriert zwei erfindungsgemäße Schraubenelemente (1) im Verbund mit u-förmigen Gabelköpfen (2) montiert mit einem Verbindungsstab (4). Die Schraubenelemente (1) besitzen einen proximalen Kopfbereich (10), welcher wenigstens abschnittsweise ein Kugelsegment aufweist, welches dazu geeignet ist, mit einen in einer Seitenansicht u-förmigen Gabelkopf (2), eine polyaxiale schwenkbare Verbindung bereitzustellen. Nach eingelegtem Verbindungsstab (4) und fixiertem Stellmittel (3) sind die Schraubenelemente (1) winkelstabil miteinander verbunden. Sie bilden eine rigide Fixierung, wie sie beispielsweise bei Wirbelsäuleneingriffen zum Einsatz kommt.

## Patentansprüche

1. Schraubenelement (1) für die Fixation von Knochenkomponenten und Knochenfragmenten bestehend aus einem Schaft (11) mit einem Knochengewinde (12) und eine sich am Schaft (11) entlang erstreckende longitudinale Zentralachse (103) und sich dadurch eine distale (102) und eine proximale (101) Richtung definiert, und das Schraubenelement (1) zusätzlich einen Kopf (10), einen Halsbereich (20) aufweist und eine Werkzeugansatzstelle (90) im Kopf (10) bereitgestellt wird, wobei das Schraubenelement eine Ti6Al4V-Legierung umfasst, **dadurch gekennzeichnet, dass** am proximalen Ende des Kopfes (10, 109) mindestens drei Ausleger-Elemente (13) befestigt sind und diese Ausleger-Elemente (13) in proximaler Richtung (101) über das proximale Ende des Kopfes (10, 109) hinausreichen und hauptsächlich entlang der longitudinalen Zentralachse (103) angeordnet sind, wobei die freien Enden (17) der Ausleger-Elemente (13) eine Ebene (108) beschreiben und das Außengewinde (12) des Schraubenelements (1) mindestens eine Gewinde-Fläche (122) besitzt, die von der Ebene (108) weggerichtet ist, und mindestens eine Gewinde-Fläche (121) besitzt, die dieser Ebene (108) zugerichtet ist und die der Ebene (108) zugerichtete Gewinde-Fläche (121) eine größere Rauigkeit aufweist als die der Ebene (108) weggerichtete Gewinde-Fläche (122).

2. Schraubenelement (1) nach vorhergehendem Anspruch, **dadurch gekennzeichnet, dass** für den 3D-Druck eine Baurichtung (105) des Schraubenelements (1) definiert ist, die in etwa der Richtung der Zentralachse (103) entspricht, und von proximal (101) nach distal (102) verläuft und die Baurichtung (105) einer Flächennormalen der Ebene (108) entspricht.

3. Schraubenelement (1) nach einem der vorhergehenden Ansprüche, **dadurch gekennzeichnet, dass** die Werkzeugansatzstelle (90) nach distaler Richtung (102) von einer Bodenwandung (93) begrenzt ist und diese Bodenwandung (93) von radial außen nach radial innen in zunehmender distaler Richtung (102) als Konus verläuft und diese Bodenwandung (93) durch einen in etwa rechtwinkligen Konuswinkel beschrieben wird.

4. Schraubenelement (1) nach einem der vorhergehenden Ansprüche, **dadurch gekennzeichnet, dass** die Werkzeugansatzstelle (90) in proximaler Richtung (101) offen ist und in einer konzentrischen kegelartigen Ausnehmung (94) mündet und diese Ausnehmung (94) einen in etwa rechtwinkligen Konuswinkel besitzt.

5. Schraubenelement (1) nach einem der vorhergehenden Ansprüche, **dadurch gekennzeichnet, dass** der Kopfbereich (10) wenigstens abschnittsweise ein Kugelsegment oder Linsensegment aufweist, welches dazu geeignet ist, mit einen in einer Seitenansicht u-förmigen Gabelkopf (2), eine polyaxiale schwenkbare Verbindung bereitzustellen.

6. Schraubenelement (1) nach einem der vorhergehenden Ansprüche, **dadurch gekennzeichnet, dass** das Außengewinde (12) sich in einen an den Halsbereich (20) angrenzenden und in distaler Richtung (102) erstreckenden proximalen Gewindebereich (30), und ein sich daran angrenzender distaler Gewindebereich (50), und wiederrum daran angrenzender distaler Spitzenbereich (60) einteilen lässt, und der distale Gewindebereich (50) in den proximalen Gewindebereich (30) in einer Übergangszone (40) ineinander übergeht, und der proximale Gewindebereich (30) mindestens einen zusätzlichen Gewindegang (31, 32) ausbildet, welcher innerhalb der Übergangszone (40) mindestens eine Schneidkante (41) ausbildet.

7. Schraubenelement (1) nach einem der vorhergehenden Ansprüche, **dadurch gekennzeichnet, dass** der distaler Spitzenbereich (60) mindestens eine Schneidkante (61) ausbildet.

8. Schraubenelement (1) nach einem der vorhergehenden Ansprüche, **dadurch gekennzeichnet, dass** das Schraubenelement (1) eine durchgängige Kanülierung (80) besitzt, die Kanülierung (80) mindestens zwei seitlich abgehende und mit der Kanülierung (80, 83) kommunizierende Öffnungen (70) besitzt und die Öffnungen (70) in einer Seitenansicht als Polygon (700) vorgesehen sind.

9. **Verfahren zum Herstellen** und Weiterbehandeln eines Schraubenelements (1) nach einem der vorhergehenden Ansprüche, umfassend die Schritte:
a. Bereitstellen von Rohmaterial-Partikeln einer Ti6A14V-Legierung;
b. Additives Aufbauen des Schraubenelements (1) mit Hilfe eines 3D-Druck-Verfahrens, welches die Rohmaterial-Partikel drei-dimensional miteinander zusammenfügt, so dass am proximalen Ende des Kopfes (10, 109) mindestens drei Ausleger-Elemente (13) befestigt sind, deren freie Enden (17) eine Ebene (108) beschreiben, und so dass das Außengewinde (12) des Schraubenelements (1) mindestens eine Gewinde-Fläche (122) besitzt, die von der Ebene (108) weggerichtet ist, und mindestens eine Gewinde-Fläche (121) besitzt, die dieser Ebene (108) zugerichtet ist und die der Ebene (108) zugerichtete Gewinde-Fläche (121) eine größere Rauigkeit aufweist als die der Ebene (108) weggerichtete Gewinde-Fläche (122);
c. Spannungsarmglühen des Schraubenelements (1) bei einer Temperatur von mindestens 500°C, jedoch nicht höher als 840°C, mit einer Haltezeit von wenigstens einer Stunde, aber kürzer als 6 Stunden;
d. Entfernen der Ausleger-Elemente (13) vom proximalen Kopfbereich (109);
e. Zweite Wärmebehandlung des Schraubenelements (1); und
f. Entfernen unvollständig gefügter Rohmaterial-Partikel.

10. Verfahren nach Anspruch 9, **dadurch gekennzeichnet, dass** beim Spannungsarmglühen des Schraubenelements (1) eine Temperatur von vorzugsweise zwischen 550°C und 800°C, vorzugsweise zwischen 550°C und 750°C, vorzugsweise zwischen 600°C und 720°C, für die Dauer der Haltezeit eingestellt ist.

11. Verfahren nach Anspruch 9, **dadurch gekennzeichnet, dass** bei der Zweiten Wärmebehandlung des Schraubenelements (1), in einer Argon-Atmosphäre, bei einer Temperatur zwischen 900°C und 940°C, vorzugsweise zwischen 910°C und 930°C stattfindet, und das Schraubenelement (1) einem Druck von mindestens 900bar, vorzugsweise von mindestens 950bar ausgesetzt ist, und für die Temperatur und Druck eine Haltezeit von mindestens einer Stunde aber weniger als 4 Stunden eingehalten wird.

12. Verfahren nach Anspruch 9, **dadurch gekennzeichnet, dass** bei der Zweiten Wärmebehandlung des Schraubenelements (1) in einer Argon-Atmosphäre bei einer Temperatur von wenigstens 920°C aber weniger als 1050°C stattfindet, und eine Haltezeit von mindestens einer Stunde aber weniger als 4 Stunden eingehalten wird.

13. Verfahren nach Anspruch 9, **dadurch gekennzeichnet, dass** bei der Zweiten Wärmebehandlung des Schraubenelements (1) bei einer Temperatur von wenigstens 820°C aber weniger als 920°C stattfindet, und eine Haltezeit von mindestens einer Stunde aber weniger als 4 Stunden eingehalten wird.

14. Verfahren nach einem der vorherigen Ansprüche 11, 12 oder 13, **dadurch gekennzeichnet, dass** das Entfernen unvollständig gefügter Rohmaterial-Partikel Folgendes umfasst:
a. Entfernen unvollständig gefügter Rohmaterial-Partikel mit Hilfe eines mechanisch-abtragenden Verfahrens, und
b. Entfernen unvollständig gefügter Rohmaterial-Partikel mit Hilfe eines chemisch-abtragenden Verfahrens.

15. Verfahren nach Anspruch 14, **dadurch gekennzeichnet, dass** das mechanisch-abtragende Verfahren mit Hilfe eines Strahlmittels, bei einem Mindestapplikationsdruck von 2bar, und einer Mindestapplikationsdauer von 5min durchgeführt wird, wobei scharfkantiges Strahlmittel, wie beispielsweise Korund, zum Einsatz kommt, und das Strahlmittel eine Korngröße zwischen 0,05mm und 0,25mm, vorzugsweise jedoch eine Korngröße zwischen 0,07mm und 0,15mm aufweist.

16. Verfahren nach Anspruch 14, **dadurch gekennzeichnet, dass** das mechanisch-abtragende Verfahren mit Hilfe eines Grobgleitschleifen- oder Vibrationsschleifverfahrens durchgeführt wird.

17. Verfahren nach Anspruch 14, **dadurch gekennzeichnet, dass** das chemisch-abtragende Verfahren mit Hilfe eines Beiz- oder Ätzvorgangs durchgeführt wird.

18. Verfahren nach Anspruch 17, **dadurch gekennzeichnet, dass** das chemisch-abtragende Verfahren elektro-galvanisch und/oder durch mechanisch induzierte Schwingungen unterstützt wird.

19. Verfahren nach einem der vorhergehenden Ansprüche 9-18, **dadurch gekennzeichnet, dass** zum Abschluss des Verfahrens die Oberflächen des Schraubenelements (1) mit Hilfe eines Strahlmittels verfestigt werden, wobei ein Mindestapplikationsdruck von 2bar, und einer Mindestapplikationsdauer von 5min eingehalten werden, wobei kugelartiges Strahlmittel zum Einsatz kommt, wie beispielsweise Keramikkugeln, und das Strahlmittel eine Korngröße zwischen 0,05mm und 0,25mm, vorzugsweise jedoch eine Korngröße zwischen 0,07mm und 0,15mm aufweist.

## Claims

1. A screw element (1) for the fixation of bone components and bone fragments consisting of a shaft (11) with a bone thread (12) and a longitudinal central axis (103) extending along the shaft (11) and thereby defining a distal (102) and a proximal (101) direction, and the screw element (1) further comprises a head (10), and a neck area (20) and a tool attachment point (90) is provided in the head (10), wherein the screw element comprises a Ti6Al4V alloy, **characterized in that** at least three cantilever elements (13) are attached to the proximal end of the head (10, 109) and said cantilever elements (13) extend in proximal direction (101) beyond the proximal end of the head (10, 109) and are positioned mainly along the longitudinal central axis (103), wherein the free ends (17) of the cantilever elements (13) describe a plane (108) and the outer thread (12) of the screw element (1) has at least one thread surface (122) which is directed away from the plane (108) and at least one thread surface (121) that faces this plane (108), and the surface (121) facing the plane has a greater roughness than the thread surface (122) directed away.

2. The screw element (1) according to the preceding claim, **characterized in that** for 3D printing a building direction (105) of the screw element (1) is defined which corresponds approximately to the direction of the central axis (103) and runs from proximal (101) to distal (102) and the building direction (105) corresponds to a surface normal of the plane (108).

3. The screw element (1) according to any of the preceding claims, **characterized in that** the tool attachment point (90) is bounded in the distal direction (102) by a bottom wall (93) and said bottom wall (93) runs as a cone from radially outside to radially inside in increasing distal direction (102) and said bottom wall (93) is defined by an approximately right-angled cone angle.

4. The screw element (1) according to any of the preceding claims, **characterized in that** the tool attachment point (90) is open in the proximal direction (101) and opens into a concentric cone-like recess (94) and this recess (94) has an approximately right-angled cone angle.

5. The screw element (1) according to any of the preceding claims, **characterized in that** the head area (10) comprises, at least in sections, a ball segment or lens segment, said head area is configured to provide a polyaxially pivotable connection with a fork head (2) that is u-shaped in a side view.

6. The screw element (1) according to any of the preceding claims, **characterized in that** the outer thread (12) can be zoned into a proximal thread area (30) adjacent to the neck area (20) and extending in the distal direction (102), and a distal thread area (50) adjacent thereto, and a distal tip area (60) adjacent thereto, and the distal thread area (50) merges into the proximal thread area (30) in a transition zone (40), and the proximal thread area (30) forms at least one further course of thread (31, 32) forming at least one cutting edge (41) within the transition zone (40).

7. The screw element (1) according to any of the preceding claims, **characterized in that** the distal tip area (60) forms at least one cutting edge (61).

8. The screw element (1) according to any of the preceding claims, **characterized in that** the screw element (1) has a continuous cannula(80), the cannula (80) has at least two laterally extending openings (70) being connected with the cannula (80, 83), and the openings (70) are provided as a polygon (700) in a side view.

9. A process for manufacturing and further processing a screw element (1) according to any of the preceding claims, comprising the steps:
a. Providing raw material particles of a Ti6Al4V alloy.
b. Additive building of the screw element (1) by means of a 3D printing process, which joins the raw material particles together in three dimensions so that at least three cantilever elements (13) are attached to the proximal end of the head (10, 109), the free ends (17) of which describe a plane (108), and so that the external thread (12) of the screw element (1) has at least one thread surface (122) that is directed away from the plane (108), and has at least one thread surface (121) that faces this plane (108), and the surface (121) that faces the plane (108) has a greater roughness than the surface (122) directed away from the plane (108).
c. Stress relief heat treatment of the screw element (1) at a temperature of at least 500°C, but not greater than 840°C, with a hold time of at least one hour, but less than 6 hours;
d. Removing the cantilever elements (13) from the proximal head area (109);
e. Second heat treatment of the screw element (1); and
f. Removing incompletely joined raw material particles.

10. The process according to claim 9, **characterized in that** during the stress relief heat treatment of the screw element (1) a temperature of preferably between 550°C and 800°C, preferably between 550°C and 750°C, preferably between 600°C and 720°C, is set for the duration of the hold time.

11. The process according to claim 9, **characterized in that** during the second heat treatment of the screw element (1), an argon atmosphere and a temperature between 900°C and 940°C, preferably between 910°C and 930°C, is applied, and the screw element (1) is exposed to a pressure of at least 900 bar, preferably at least 950 bar, and for the temperature and the pressure a hold time of at least one hour but less than 4 hours is applied.

12. The process according to claim 9, **characterized in that** during the second heat treatment of the screw element (1), an argon atmosphere at a temperature of at least 920°C but less than 1050°C is applied, and a hold time of at least one hour but less than 4 hours is applied.

13. The process according to claim 9, **characterized in that** during the second heat treatment of the screw element (1) a temperature of at least 820°C but less than 920°C is applied, and a hold time of at least one hour but less than 4 hours is applied.

14. The process of any of the preceding claims 11, 12 or 13, **characterized in that** removing incompletely joined raw material particles comprises:
a. Removing incompletely joined raw material particles by means of a mechanical removal process, and
b. Removing incompletely joined raw material particles by means of a chemical removal process.

15. The process according to claim 14, **characterized in that** the mechanical removal process is conducted by means of blast means at a minimum application pressure of 2 bar and a minimum application duration of 5 min, wherein sharp-edged blast means, such as corundum, is used, and the blast means comprises a particle size between 0.05 mm and 0.25 mm, but preferably a particle size between 0.07 mm and 0.15 mm.

16. The process according to claim 14, **characterized in that** the mechanical removal process is conducted by means of a coarse vibratory finishing or vibratory finishing process.

17. The process according to claim 14, **characterized in that** the chemical removal process is conducted by means of a staining or etching treatment.

18. The process according to claim 17, **characterized in that** the chemical removal process is supported electro-galvanically and/or by mechanically induced vibrations.

19. The process according to any of the preceding claims 9-18, **characterized in that**, at the end of the process, the surfaces of the screw element (1) are solidified by means of a blasting abrasive, wherein a minimum application pressure of 2 bar and a minimum application duration of 5 min are applied, wherein spherical blasting abrasives are used, such as ceramic balls, and the blasting abrasive has a particle size between 0.05 mm and 0.25 mm, but preferably a particle size between 0.07 mm and 0.15 mm.

## Revendications

1. Élément fileté (1) pour la fixation de composants osseux et de fragments osseux composé d'une tige (11) avec un filetage osseux (12) et un axe central longitudinal (103) s'étendant le long de la tige (11) et définissant ainsi une direction distale (102) et une direction proximale (101), et l'élément fileté (1) présentant en outre une tête (10), une zone de cou (20) et un point d'attache de l'outil (90) dans la tête (10), l'élément fileté comprenant un alliage Ti6A14V , **caractérisé en ce qu'**au moins trois éléments en porte-à-faux (13) sont fixés à l'extrémité proximale de la tête (10, 109) et ces éléments en porte-à-faux (13) s'étendent dans la direction proximale (101) au-delà de l'extrémité proximale de la tête (10, 109) et sont disposées principalement le long de l'axe central longitudinal (103), les extrémités libres (17) des éléments en porte-à-faux (13) décrivant un plan (108) et le filetage extérieur (12) de l'élément fileté (1) ayant au moins une surface filetée (122) orientée à l'opposé du plan (108) et ayant au moins une surface filetée (121) orientée vers ce plan (108) et la surface filetée (121) orientée vers le plan (108) ayant une rugosité supérieure à celle de la surface filetée (122) orientée à l'opposé du plan (108).

2. Élément fileté (1) selon la revendication précédente, **caractérisé en ce que**, pour l'impression 3D, une direction de construction (105) de l'élément fileté (1) est définie, qui correspond approximativement à la direction de l'axe central (103), et s'étend de proximal (101) à distal (102), et la direction de construction (105) correspond à une normale à la surface du plan (108).

3. Élément fileté (1) selon l'une des revendications précédentes, **caractérisé en ce que** le point d'attache de l'outil (90) est limité en direction distale (102) par une paroi de fond (93) et cette paroi de fond (93) s'étend sous forme de cône de l'extérieur radialement vers l'intérieur radialement dans une direction distale croissante (102) et cette paroi de fond (93) est décrite par un angle de cône à peu près à angle droit.

4. Élément fileté (1) selon l'une des revendications précédentes, **caractérisé en ce que** point d'attache de l'outil (90) est ouvert dans la direction proximale (101) et débouche dans un évidement conique concentrique (94) et cet évidement (94) présente un angle de cône à peu près rectangulaire.

5. Élément fileté (1) selon l'une des revendications précédentes, **caractérisé en ce que** la zone de tête (10) présente au moins par sections un segment sphérique ou un segment de lentille, qui est adapté pour fournir une connexion pivotante polyaxiale avec une tête de fourche (2) en forme de U dans une vue latérale.

6. Élément fileté (1) selon l'une des revendications précédentes, **caractérisé en ce que** le filetage extérieur (12) peut être divisé en une zone de filetage proximale (30) adjacente à la zone de cou (20) et s'étendant dans la direction distale (102), et une zone de filetage distale (50) adjacente à celle-ci, et à nouveau une zone de pointe distale (60) adjacente à celle-ci, et la zone filetée distale (50) se raccorde à la zone filetée proximale (30) dans une zone de transition (40), et la zone filetée proximale (30) forme au moins un filet supplémentaire (31, 32) qui forme au moins une arête de coupe (41) à l'intérieur de la zone de transition (40).

7. Élément fileté (1) selon l'une des revendications précédentes, **caractérisé en ce que** la zone de pointe distale (60) forme au moins une arête de coupe (61).

8. Élément fileté (1) selon l'une des revendications précédentes, **caractérisé en ce que** l'élément fileté (1) possède une canulation (80) continue, la canulation (80) possède au moins deux ouvertures (70) se développant latéralement et communiquant avec la canulation (80, 83) et les ouvertures (70) sont prévues sous forme de polygone (700) dans une vue latérale.

9. **Procédé de fabrication** et de traitement ultérieur d'un élément fileté (1) selon l'une quelconque des revendications précédentes, comprenant les étapes suivantes :
a. fournir des particules de matière première d'un alliage Ti6A14V ;
b. construction additive de l'élément fileté (1) à l'aide d'un procédé d'impression 3D qui assemble les particules de matière première en trois dimensions, de sorte qu'au moins trois éléments en porte-à-faux (13) sont fixés à l'extrémité proximale de la tête (10, 109), dont les extrémités libres (17) décrivent un plan (108), et de sorte que le filetage extérieur (12) de l'élément fileté (1) possède au moins une surface filetée (122) orientée à l'opposé du plan (108) et au moins une surface filetée (121) orientée vers ce plan (108) et dont la surface filetée (121) orientée vers le plan (108) présente une rugosité supérieure à celle de la surface filetée (122) orientée à l'opposé du plan (108) ;
c. recuit de détente de l'élément fileté (1) à une température d'au moins 500 °C, mais ne dépassant pas 840 °C, avec un temps de maintien d'au moins une heure, mais inférieur à 6 heures ;
d. retrait des éléments en porte-à-faux (13) de la zone de tête proximale (109) ;
e. deuxième traitement thermique de l'élément fileté (1) ; et
f. élimination des particules de matière première assemblées de manière incomplète.

10. Procédé selon la revendication 9, **caractérisé en ce que**, lors du recuit de détente de l'élément fileté (1), une température de préférence comprise entre 550 °C et 800 °C, de préférence entre 550 °C et 750 °C, de préférence entre 600 °C et 720 °C, est réglée pour la durée du temps de maintien.

11. Procédé selon la revendication 9, **caractérisé en ce que**, le deuxième traitement thermique de l'élément fileté (1), a lieu dans une atmosphère d'argon, à une température comprise entre 900 °C et 940 °C, de préférence entre 910 °C et 930 °C, et l'élément fileté (1) est soumis à une pression d'au moins 900 bars, de préférence d'au moins 950 bars, et un temps de maintien d'au moins une heure mais de moins de 4 heures est respecté pour la température et la pression.

12. Procédé selon la revendication 9, **caractérisé en ce que** le deuxième traitement thermique de l'élément fileté (1) a lieu dans une atmosphère d'argon à une température d'au moins 920 °C mais inférieure à 1050 °C, et un temps de maintien d'au moins une heure mais inférieur à 4 heures est respecté.

13. Procédé selon la revendication 9, **caractérisé en ce que** le deuxième traitement thermique de l'élément fileté (1) a lieu à une température d'au moins 820 °C mais inférieure à 920 °C, et un temps de maintien d'au moins une heure mais inférieur à 4 heures est respecté.

14. Procédé selon l'une quelconque des revendications précédentes 11, 12 ou 13, **caractérisé en ce que** l'élimination des particules de matière première assemblées de manière incomplète comprend :
a. élimination des particules de matière première assemblées de manière incomplète à l'aide d'un procédé d'abrasion mécanique, et
b. élimination des particules de matière première assemblées de manière incomplète à l'aide d'un procédé d'abrasion chimique.

15. Procédé selon la revendication 14, **caractérisé en ce que** le procédé d'abrasion mécanique est effectué à l'aide d'un moyen de sablage, avec une pression d'application minimale de 2 bars et une durée d'application minimale de 5 minutes, en utilisant un moyen de sablage à arêtes vives, tel que du corindon, et **en ce que** le moyen de sablage présente une granulométrie comprise entre 0,05 mm et 0,25 mm, mais de préférence une granulométrie comprise entre 0,07 mm et 0,15 mm.

16. Procédé selon la revendication 14, **caractérisé en ce que** le procédé d'abrasion mécanique est réalisé à l'aide d'un procédé de dégrossissage ou de ponçage vibratoire.

17. Procédé selon la revendication 14, **caractérisé en ce que** le procédé d'abrasion chimique est réalisé à l'aide d'un processus de décapage ou de gravure.

18. Procédé selon la revendication 17, **caractérisé en ce que** le procédé d'abrasion chimique est assisté électro-galvaniquement et/ou par des vibrations induites mécaniquement.

19. Procédé selon l'une quelconque des revendications précédentes 9 à 18, **caractérisé en ce qu'**à la fin du procédé, les surfaces de l'élément fileté (1) sont solidifiées à l'aide d'un moyen de sablage, en respectant une pression d'application minimale de 2 bars et une durée d'application minimale de 5 minutes, en utilisant un moyen de sablage sphérique, tel que des billes en céramique, et **en ce que** le moyen de sablage a une granulométrie comprise entre 0,05 mm et 0,25 mm, mais de préférence une granulométrie comprise entre 0,07 mm et 0,15 mm.
